Europäisches Patentamt

European Patent Office

Office européen des brevets

(19)

(11) **EP 1 142 560 A2**

(12) **EUROPEAN PATENT APPLICATION**

(43) Date of publication:
**10.10.2001 Bulletin 2001/41**

(51) Int Cl.[7]: **A61K 7/13**

(21) Application number: **01107830.0**

(22) Date of filing: **06.04.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **07.04.2000 JP 2000107179**

(71) Applicant: **Kao Corporation**
**Tokyo 103-8210 (JP)**

(72) Inventors:
• **Miyabe, Hajime, Kao Corporation**
**Sumida-ku, Tokyo 131-8501 (JP)**
• **Matsunaga, Kenichi, Kao Corporation**
**Sumida-ku, Tokyo 131-8501 (JP)**

(74) Representative: **HOFFMANN - EITLE**
**Patent- und Rechtsanwälte**
**Arabellastrasse 4**
**81925 München (DE)**

(54) **Hair dye composition**

(57) Provided is a hair dye composition comprising a first part containing an alkaline agent and a second part containing an oxidizing agent, wherein the first part contains (A) the alkaline agent, (B) a cationic direct dye, (C) a nonionic surfactant, (D) water, and (E) an anionic component selected from anionic surfactants and anionic polymers; and the anionic component (E) is added in an amount, relative to the cationic direct dye (B), to satisfy the following equation:

[Ion activity concentration of the anionic component / Ion activity concentration of the cationic direct dye $\leq$ 8]

(The term "ion activity concentration" as used herein means "molar concentration x ionic valence"). Since the anionic component does not disturb the dyeing of the hair with the cationic direct dye, this hair dye composition exhibits excellent dyeing power.

**EP 1 142 560 A2**

## Description

### Technical Field

The present invention relates to a two-part hair dye composition using a cationic dye as a direct dye. This composition exhibits excellent dyeing power, because the anionic component contained therein does not disturb the dyeing of the hair with the cationic direct dye.

### Background Art

Known is a two-part hair dye composition to be used by adding a direct dye to a hair bleaching composition which carries out bleaching by activating an oxidizing agent at an alkaline pH and effecting oxidation bleaching of melanin pigment. When this hair dye composition is applied to the hair of a dark color, a change in color shade of the hair before and after dyeing therewith is easily distinguishable compared with the application of a hair dye composition having no bleaching action.

In order to facilitate mixing of two parts of this composition rightly before use, mixing ease and a sufficient viscosity to wet the hair are required. For viscosity increase, a soap or anionic polymer is added. An anionic surfactant other than soap can also be added to promote dissolution of the direct dye.

An anionic component such as anionic polymer or anionic surfactant is however accompanied with the problem that when a cationic dye is added as a direct dye, the anionic component disturbs dyeing of the hair with the cationic dye, thereby lowering the dyeing power.

### Disclosure of the Invention

An object of the present invention is to provide a cationic-direct-dye-containing two-part hair dye composition which is free from disturbance, by the anionic component, of hair dyeing with the cationic direct dye, and therefore has excellent dyeing power.

The present inventors have found that the above-described problem can be prevented by adding the anionic component and cationic direct dye in suitable amounts satisfying a predetermined requirement.

In one aspect of the present invention, there is thus provided a hair dye composition comprising a first part containing an alkaline agent and a second part containing an oxidizing agent, wherein the first part contains (A) the alkaline agent, (B) a cationic direct dye, (C) a nonionic surfactant, (D) water, and (E) an anionic component selected from anionic surfactants and anionic polymers; and the anionic component (E) is added in an amount, relative to the cationic direct dye (B), to satisfy the following equation:

$$[\text{Ion activity concentration of the anionic component} / \text{Ion activity concentration of the cationic direct dye} \leq 8]$$

(The term "ion activity concentration" as used herein means "molar concentration x ionic valence")

In another aspect of the present invention, there is also provided a hair dyeing method which comprises applying the above-described hair dye composition to the hair at 15 to 45°C, allowing the composition to act on the hair for 1 to 50 minutes, washing the thus-treated hair and then drying.

### Best Mode for Carrying out the Invention

In the present invention, disturbance, by the anionic component, of hair dyeing with the cationic dye is prevented by adjusting the ion activity concentration ratio (E)/(B) of the anionic component (E) to the cationic direct dye (B) in the first part (which ratio will hereinafter be called "ion activity concentration ratio" simply) to 8 or less. Ratios of 6 or less are more preferred.

Examples of the anionic component (E) to be incorporated in the first part include anionic surfactants, e.g., salts (soaps) of a fatty acid such as oleic acid, stearic acid, isostearic acid, lauric acid, myristic acid or behenic acid, alkyl sulfate ester salts such as sodium laurylsulfate, alkyl ether sulfate ester salts such as sodium polyoxyethylene lauryl sulfate, N-acylated glutamate salts such as monosodium N-lauroylglutamate, and N-acylated sarcosinate salts, alkylether phosphate ester salts, alkyl ether sulfosuccinate salts and alkylbenzenesulfonate salts; and anionic polymers such as carboxymethyl cellulose, carboxyvinyl polymers, polyacrylic acid and polymethacrylic acid and derivatives thereof.

As the anionic component (E), one or more can be used and it is incorporated to give an ion activity concentration ratio of 8 or less.

Examples of the cationic direct dye (B), that is, a direct dye containing a quaternary nitrogen atom include any conventionally known cationic direct dye such as Basic Blue 7 (C.I. 42595), Basic Blue 26 (C.I. 44045), Basic Blue 93, Basic Blue 99 (C.I. 56059), Basic Blue 117, Basic Violet 10 (C.I. 45170), Basic Violet 14 (C.I. 42515), Basic Brown 16 (C.I. 12250), Basic Brown 17 (C.I. 12251), Basic Red 2 (C.I. 50240), Basic Red 22 (C.I. 11055), Basic Red 76 (C.I. 12245), Basic Red 118 (C.I. 12251:1), Basic Yellow 57 (C.I. 12719), Basic Yellow 21 (C.I. 48060) and Basic Yellow 28 (C.I. 48054); cationic direct dyes as described in Japanese Patent Publication No. Sho 58-2204 or Japanese Patent Application Laid-Open No. Hei 9-118832 which contains a quaternized nitrogen atom at the side chain of the aromatic group; and cationic direct dyes as described in Japanese Patent Application Laid-Open No. Hei 10-182378, each containing a quaternized nitrogen atom which may be nonlocalized and contains an azo bond, methine bond or azamethine bond.

As the cationic direct dye (B), one or more can be incorporated in the first part. It is preferably added in an amount of 0.01 to 10 wt.%, particularly 0.05 to 5 wt.%, which amount is presumed to satisfy the ion activity concentration ratio of 8 or less.

Examples of the alkaline agent (A) to be incorporated in the first part include alkanolamines such as monoethanolamine and isopropanolamine, guanidium salts such as guanidine carbonate and ammonia, with alkanolamines, particularly monoethanolamine being preferred. As the alkaline agent, one or more can be incorporated in the first part. From the viewpoints of sufficient hair dyeing effects and low irritation to the scalp, it is preferably added in an amount of 0.1 to 10 wt.%, more preferably 0.5 to 5 wt.%, especially 1 to 3 wt.% in the whole composition as a mixture of the first part and second part.

As the nonionic surfactant (C), one or more can be incorporated in the first part. Particularly preferred is the incorporation of at least two nonionic surfactants different in HLB, that is, (c1) a hydrophilic nonionic surfactant having an HLB of 10 to 20 and (c2) a lipophilic nonionic surfactant having an HLB of 1 to 10.

Examples of hydrophilic nonionic surfactant (c1) include polyoxyethylene alkyl ethers such as polyoxyethylene octyl dodecyl ether, polyoxyethylene oleyl ether, polyoxyethylene tridecyl ether, polyoxyethylene lauryl ether and polyoxyethylene isostearyl ether, and alkyl glycosides, each having an HLB of 10 to 20.

Examples of the lipophilic nonionic surfactant (c2) include the polyoxyethylene alkyl ethers which are exemplified above but have an HLB of 1 to 10, and polyoxyethylene polypropylene alkyl ethers, alkyl glyceryl ethers, alkyl glyceryl pentaerythrityl ether, alkyldiglyceryl ether and alkyltriglyceryl ether each having an HLB 1 to 10. Out of them, isostearyl glyceryl ether, isostearyl diglyceryl ether and isostearyl glyceryl pentaerythrityl ether are particularly preferred.

The nonionic surfactant (c) is preferably added in an amount of 1 to 60 wt.%, more preferably 5 to 50 wt.%, especially 10 to 40 wt.% in the whole composition as a mixture of the first and second parts. When the above-described components (c1) and (c2) are incorporated at a weight ratio of 1/10 to 10/1 so as to give an average HLB of 8 to 12, preferably 9 to 11, even in the case where both of the first and second parts are in the liquid form, thickening occurs by mixing, which prevents sagging of the resulting composition upon application to the hair. The term "HLB" as used herein means that determined by the Griffin's method, while the term "liquid form" as used herein means a state having a viscosity of 3000 mPa·s or less, preferably 1000 mPa·s or less as measured at 25°C by a B type rotaviscometer. The mixture of the first and second parts preferably has a viscosity of 3000 mPa·s or greater, with 5000 mPa·s or greater being particularly preferred. Viscosity as used herein means a value measured after the rotation of 12 rpm for 1 minute by using rotor No.3.

The hair dye composition of the present invention contains water (D) and its content is preferably 20 wt.% or greater.

Examples of the oxidizing agent to be incorporated in the second part include hydrogen peroxide, urea peroxide, alkali metal bromates, and alkali metal salts of a peracid (perbromates, persulfates and perborates), with hydrogen peroxide being particularly preferred. The amount of hydrogen peroxide added, for example, as the oxidizing agent ranging from 1 to 12 wt.% is preferred for sufficient bleaching and hair dyeing effects and low irritation to the scalp.

An oxidation dye can be added further to the hair dye composition of the present invention. As the oxidation dye, precursors and couplers ordinarily employed for an oxidizing type hair dye composition can be used.

In addition to the above-described components, those ordinarily employed in the field of cosmetics can be added to the hair dye composition of the present invention. Examples of such an optional component include natural or synthetic polymer compounds, oils and fats, hydrocarbons, higher alcohols, monohydric or polyhydric alcohols, silicone derivatives, amine oxides, amino acid derivatives, protein derivatives, antiseptics, sequestering agents, antioxidants, stabilizers for hydrogen peroxide, plant extracts, vitamins, pigments, ultraviolet absorbers and perfumes.

It is preferred to use the hair dye composition of the present invention by mixing, upon application, the first part and second part at a ratio of 2:1 to 1:3 (weight ratio). These first and second parts may each be obtained in any one of the forms such as liquid, emulsion, cream, gel, paste or mousse. They may also be obtained as an aerosol form.

Upon application, the hair dye composition of the present invention preferably has a pH of 8 to 12, especially 8.5 to 11. At a pH less than 8, the advantages of the present invention are not available sufficiently. On the other hand, pHs exceeding 12 are not suited for practical use because of strong irritation to the scalp.

The hair dye composition of the present invention is used, for example, by applying it to the hair at 15 to 45°C, allowing it to act on the hair for 1 to 50 minutes, preferably 3 to 20 minutes, washing the hair and drying the same.

- Examples -

Examples 1,2 and Comparative Example 1

The hair dyeing compositions shown in Table 1 were prepared and their dyeing properties were evaluated.

(Evaluating method)

Four hair bundles, each having a length of 15 cm and weight of 20 g, were made using the brown hair of a German. The hair dye compositions (having one part and second part mixed at a weight ratio of 1:1) of Examples 1 and 2, and Comparative Example 1, each 20 g, were spread well over the three bundles, respectively. After they were allowed to stand for 20 minutes in a thermostat of 30°C, the hair bundles were rinsed with warm water of 40°C, treated with a shampoo and rinse and then dried. The reddish degree of these hair bundles compared with the untreated hair bundle was evaluated by 10 panelists based on the below-described standards. The compositions were each evaluated by a total point.

(Evaluation standards)

The reddish degree of the hair was strong         3
The reddish degree of the hair was slightly strong       2
The reddish degree of the hair was slight       1
The reddish degree of the hair was scarce       0

Table 1

| | | Example | | Comp. Ex. |
|---|---|---|---|---|
| | | 14 | 2 | 1 |
| First part | Basic Red 76 | 0.4 | 0.4 | 0.4 |
| | 28 wt.% Aqueous ammonia | 5 | 5 | 5 |
| | Monoethanolamine | 2 | 2 | 2 |
| | Cetanol | 8.5 | 8.5 | 8.5 |
| | Polyoxyethylene (40) cetyl ether | 3 | 3 | 3 |
| | Polyoxyethylene (2) cetyl ether | 3.5 | 3.5 | 3.5 |
| | Stearyl trimethylammonium chloride | 2 | 2 | 2 |
| | Sodium laurylsulfate | - | 2.5 | 4 |
| | Liquid paraffin | 0.5 | 0.5 | 0.5 |
| | Tetrasodium ethylenediaminetetraacetate | 0.1 | 0.1 | 0.1 |
| | Perfume | q.s. | q.s. | q.s. |
| | Ammonium chloride | Amount to adjust pH to 10 | Amount to adjust pH to 10 | Amount to adjust pH to 10 |
| | Water | Balance | Balance | Balance |
| | Ion activity concentration ratio | 0 | 5.8 | 9.2 |
| Second part | 35 wt.% Aqueous hydrogen peroxide | 17.1 | 17.1 | 17.1 |
| | Methyl paraben | 0.1 | 0.1 | 0.1 |
| | Phosphoric acid | Amount to adjust pH to 3.5 | Amount to adjust pH to 3.5 | Amount to adjust pH to 3.5 |
| | Water | Balance | Balance | Balance |
| | Evaluation of dyeing properties | 28 | 23 | 9 |
| (The amount for each component is represented by wt.%.) | | | | |

Example 3

The hair dye composition as described below was prepared (used after mixing the first and second parts at the same amount)


    First part                                                              (wt.%)

Orange dye of the following structural formula: 0.6

| | |
|---|---|
| 28 wt.% Aqueous ammonia | 5 |
| Monoethanolamine | 2 |
| Oleyl alcohol | 5 |
| Oleic acid | 3 |
| Polyoxyethylene (30) oleocetyl ether | 7 |
| Polyoxyethylene (12) lauryl ether | 8 |
| Polyoxyethylene (3.5) decyl ether ("Mergital BL309", trade name; product of Henkel KGaA) | 22 |
| "Merquat 100" (trade name; a 40 wt.% aqueous solution) | 7.5 |
| Tetrasodium ethylenediaminetetraacetate | 0.1 |
| Perfume | q.s. |
| Ammonium chloride | Amount to adjust pH to 10 |
| Water | Balance |

(Ion activity concentration ratio = 0)

| Second part | (wt.%) |
|---|---|
| 35 wt.% Aqueous hydrogen peroxide | 17.1 |
| Methylparaben | 0.1 |
| Phosphoric acid | Amount to adjust pH to 3.5 |
| Water | Balance |

Example 4

The hair dye composition as described below was prepared (used after mixing the first and second parts at the same amount)

```
First part                                          (wt.%)

   Yellow dye of the following structural formula:     0.5
```

$$H_3C-\overset{+}{N}\!\!\!\!\!\!\!\!\!\bigcirc\!\!\!\!\!\!-\overset{\underset{\displaystyle H}{|}}{C}=N-\overset{\underset{\displaystyle CH_3}{|}}{N}-\bigcirc\!\!\!\!\!-OCH_3 \qquad CH_3SO_4^-$$

```
   Para-phenylenediamine                               0.4

   Resorcin                                            0.35

   28 wt.% Aqueous ammonia                              5

   Monoethanolamine                                     2

   Cetanol                                              3

   Polyoxyethylene (30) oleocetyl ether               4.2

   Polyoxyethylene (12) lauryl ether                  4.8

   Polyoxyethylene (3.5) decyl ether

      ("Mergital BL309", trade name; product of

       Henkel KGaA)                                   13.2

   "Merquat 100" (trade name; a 40 wt.% aqueous

       solution)                                       7.5

   Sodium sulfite                                      0.5

   Ascorbic acid                                       0.1

   Tetrasodium ethylenediaminetetraacetate            0.1

   Perfume                                             q.s.

   Ammonium chloride              Amount to adjust pH to 10
```

| | |
|---|---|
| Water | Balance |

(Ion activity concentration ratio = 0)

| Second part | (wt.%) |
|---|---|
| 35 wt.% Aqueous hydrogen peroxide | 17.1 |
| Methylparaben | 0.1 |
| Phosphoric acid | Amount to adjust pH to 3.5 |
| Water | Balance |

## Claims

1.  A hair dye composition comprising a first part containing an alkaline agent and a second part containing an oxidizing agent, wherein the first part contains (A) the alkaline agent, (B) a cationic direct dye, (C) a nonionic surfactant, (D) water, and (E) an anionic component selected from anionic surfactants and anionic polymers; and the anionic component (E) is added in an amount, relative to the cationic direct dye (B), to satisfy the following equation:

    [Ion activity concentration of the anionic component / Ion activity concentration of the cationic direct dye $\leq 8$]

    (The term "ion activity concentration" as used herein means "molar concentration x ionic valence")

2.  A hair dyeing method which comprises applying a hair dye composition as claimed in claim 1 to the hair at 15 to 45°C, allowing the composition to act on the hair for 1 to 50 minutes, washing the thus-treated hair and then drying.